# EUROPEAN PATENT APPLICATION

(11) **EP 4 649 902 A1**
(43) Date of publication of application: **19.11.2025**
(21) Application number: 25176030.2
(22) Date of filing: 13.05.2025
(51) Int. Cl.: A61B 17/30, A61B 17/34, A61B 17/32, A61B 90/00

(54) **SUBCUTANEOUS IMPLANTABLE MEDICAL DEVICE EXTRACTION TOOL**

(30) Priority: 14.05.2024 US 202463647343 P; 07.05.2025 US 202519201640
(71) Applicant: Pacesetter, Inc., Sylmar, CA 91342 (US)
(72) Inventor: Hawkins, Rodney, Santa Clarita, CA (US); Hanze, Zachary, Porter Ranch, CA (US); Park, Seung Dean, Santa Clarita, CA (US); Ryu, Kyungmoo, Los Angelos, CA (US)
(74) Representative: Barker Brettell Sweden AB

(57) **Abstract**

An extraction tool (100) includes a handle (102) and a tray (104) that extends from the handle (102). The tray (104) is configured to be advanced into a subcutaneous region of a patient. The tray (104) includes a platform (110) and a raised rim (112) that projects above a top surface (114) of the platform (110). The top surface (114) of the platform (110) and the raised rim (112) define a cavity (116) configured to receive and contain a subcutaneous implantable medical device (S-IMD) (200, 250) therein. The handle (102) is configured to be manipulated by an operator to withdraw the extraction tool (100) from the patient with the S-IMD (200, 250) on the tray (104).

## Description

### TECHNICAL FIELD

Embodiments of the present disclosure relate generally to tools for removing implantable medical devices (IMDs) from patients.

### BACKGROUND

Some IMDs include circuitry that monitors a patient's heart rhythm to detect arrythmias, such as ventricular tachycardia, ventricular fibrillation, and/or atrial fibrillation. Some of these IMDs may be subcutaneous IMDs (S-IMDs) designed to be implanted below the skin but outside of cardiac tissue and blood vessels, such as along an exterior of the rib cage. A physician may decide to remove an implanted S-IMD from a patient for various reasons. For example, the S-IMD may have reached the end of its operating lifetime, the S-IMD may have moved from a target implant location, the S-IMD may have a fault, the patient's condition may have changed since implanting the S-IMD so the S-IMD is no longer effective or needed, or the like.

A known procedure to extract an S-IMD from a patient involves the physician forming an incision in the skin proximate to the S-IMD via a scalpel. The physician then advances a forceps (e.g., tweezers) through the to access the implanted S-IMD. The physician manually manipulates the forceps to grip and pull the SIMD from a subcutaneous implant pocket in the patient. However, it may be difficult for the physician to sufficiently grasp and pull the SIMD with the forceps to extract the S-IMD from the patient. For example, the SIMD may have a relatively smooth outer surface so the forceps may be prone to slip along the outer surface when the forceps is withdrawn. Furthermore, tissue encapsulation around the S-IMD may hold the S-IMD in the implant pocket and increase the force necessary to extricate the S-IMD from the patient. These complications may undesirably increase the time and manual effort (e.g., energy) expended during the extraction procedure. These complications may frustrate the physician and/or the patient. Furthermore, to avoid slippage the physician may apply more force on the forceps, which could risk damaging the S-IMD and/or inflicting trauma on the patient tissue.

A need remains for an extraction tool that is designed to relatively easily and efficiently remove an S-IMD from a patient. A need remains for an extraction tool that avoids the issues with using conventional forceps to perform the task.

### SUMMARY

In accordance with embodiments herein, an extraction tool is provided that includes a handle and a tray that extends from the handle. The tray is configured to be advanced into a subcutaneous region of a patient. The tray includes a platform and a raised rim that projects above a top surface of the platform. The top surface of the platform and the raised rim define a cavity configured to receive and contain a subcutaneous implantable medical device (S-IMD) therein. The handle is configured to be manipulated by an operator to withdraw the extraction tool from the patient with the S-IMD on the tray.

In an embodiment, the raised rim may be located at a distal edge of the platform and may extend along at least a portion of opposite first and second side edges of the platform.

In an embodiment, the raised rim extends along a full perimeter of the platform and surround the top surface of the platform.

In an embodiment, a distal end of the tray has a tapered tip. The platform and raised rim may be located between the handle and the tapered tip.

In an embodiment, the tray extends from a distal end of the handle, and a proximal end of the handle has a tapered tip. The tapered tip, at the distal end of the tray and/or at the proximal end of the handle, may have a blunt end for blunt dissection of patient tissue from the S-IMD.

In an embodiment, the raised rim has a depression at a distal end of the tray configured to accommodate a projection that extends from a housing of the S-IMD.

In an embodiment, the raised rim at a distal edge of the platform is curved along a height of the raised rim to form a shelf that at least partially overhangs the platform and defines a pocket. The pocket may be configured to receive a distal end of the S-IMD therein.

In an embodiment, length and width dimensions of the platform within the cavity are based on length and width dimensions of the S-IMD. A shape of the cavity defined by the raised rim may correspond to a shape of the S-IMD. For example, the platform within the cavity may have a stadium shape.

In accordance with embodiments herein, an extraction tool is provided that includes a handle and a tray. The handle includes a proximal end and a distal end. The proximal end of the handle has a tapered tip. The tray extends from the distal end of the handle and is configured to be advanced into a subcutaneous region of a patient. The tray includes a platform and a raised rim that projects above a top surface of the platform. The raised rim extends along a full perimeter of the platform and surrounds the top surface of the platform to define a cavity configured to receive and contain an S-IMD therein. The handle is configured to be manipulated by an operator to withdraw the extraction tool from the patient with the S-IMD on the tray.

In an embodiment, the handle includes a proximal end and a distal end. The proximal end of the handle has a tapered tip. The tray extends from the distal end of the handle. The raised rim extends along a full perimeter of the platform and surrounds the top surface of the platform to define the cavity therein.

In an embodiment, the handle linearly extends from the proximal end to the distal end.

In an embodiment, the tapered tip of the handle has a blunt end for blunt dissection of patient tissue from the S-IMD. The tapered tip of the handle may be a first tapered tip, and the tray may include a second tapered tip at a distal end of the tray.

In an embodiment, the top surface of the platform is planar, and a first segment of the raised rim at a distal edge of the platform extends a greater height from the top surface than a second segment of the raised rim that is spaced apart from the distal edge of the platform.

In an embodiment, the cavity has an oblong shape defined by the raised rim.

In accordance with embodiments herein, a hinged extraction tool is provided that includes a first arm and a second arm extending from a hinge. Each of the first and second arms defines a channel along a length of the respective arm. The channels are configured to receive portions of a housing of an S-IMD therein to enhance a grip of the hinged extraction tool on the housing as an operator compresses the first and second arms towards each other.

In an embodiment, the channels are windows that fully extend through a thickness of the first and second arms.

In another embodiment, the channels are grooves defined along inner surfaces of the first and second arms.

In accordance with embodiments herein, a hinged extraction tool is provided that includes a first arm and a second arm extending from a hinge. The first arm includes a first tab at a distal end of the first arm, and the second arm includes a second tab at a distal end of the second arm. The first tab extends into a gap defined between the first and second arms in a direction towards the second arm, and the second tab extends into the gap towards the first arm. The first and second tabs and the first and second arms define a cavity configured to receive an S-IMD therein. The first and second arms are configured to be pressed towards one another by an operator to grip the S-IMD.

### BRIEF DESCRIPTION OF THE DRAWINGS

Figure 1 illustrates an extraction tool formed in accordance with an example embodiment.
Figure 2 shows an S-IMD implanted in a subcutaneous pocket of a patient below the skin of the patient.
Figure 3 shows a portion of the extraction tool at a first advancement position as the extraction tool is inserted through an incision into the patient.
Figure 4 shows the extraction tool at a second advancement position as the extraction tool is moved along an underside of the S-IMD.
Figure 5 shows the extraction tool at a withdrawal position as the extraction tool is being removed from the patient.
Figure 6 is a cross-sectional view of a portion of a tray of the extraction tool according to another example embodiment.
Figure 7 shows the extraction tool according to an example embodiment in which a raised rim of the tray fully surrounds a platform top surface.
Figure 8 shows the extraction tool according to another example embodiment in which a handle of the extraction tool includes a tapered tip.
Figure 9 shows the extraction tool according to an example embodiment in which the raised rim fully surrounds an S-IMD disposed within a cavity of the tray.
Figure 10 shows the tray of the extraction tool according to an example embodiment in which the raised rim defines a depression at a distal end of the tray.
Figure 11 shows the tray of Figure 10 with an S-IMD located in the cavity.
Figure 12 is an elevation view of the extraction tool according to a first indicator embodiment.
Figure 13 illustrates a side view of the extraction tool shown in Figure 12.
Figure 14 is an elevation view of the extraction tool according to a second indicator embodiment.
Figure 15 illustrates a side view of the extraction tool shown in Figure 14.
Figure 16 is an exploded elevation view of a distal portion of the extraction tool according to a third indicator embodiment that is a variation of the second indicator embodiment shown in Figures 14 and 15.
Figure 17 is an elevation view of a distal portion of the extraction tool according to a fourth indicator embodiment that is another variation of the second indicator embodiment shown in Figures 14 and 15.
Figure 18 is an exploded, elevation view of the extraction tool according to a fifth indicator embodiment.
Figure 19 is a side cross-sectional view of the extraction tool shown in Figure 18.
Figure 20 illustrates a cross-sectional view of a distal portion of the extraction tool according to a sixth indicator embodiment.
Figure 21 illustrates a hinged extraction tool according to an example embodiment.
Figure 22 illustrates the hinged extraction tool according to a second hinged embodiment.
Figure 23 illustrates another hinged extraction tool according to an embodiment.
Figure 24 shows a distal section of the hinged extraction tool of Figure 23 gripping an S-IMD.

### DETAILED DESCRIPTION

It will be readily understood that the components of the embodiments as generally described and illustrated in the figures herein, may be arranged and designed in a wide variety of different configurations in addition to the described example embodiments. Thus, the following more detailed description of the example embodiments, as represented in the figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that, other methods may be used, in accordance with an embodiment herein.

Embodiments of the extraction tool described herein may be implemented in connection with one or more implantable medical devices (IMDs). Non-limiting examples of IMDs include neurostimulator devices, implantable monitoring and/or therapy devices, catheters, and/or alternative implantable medical devices. At least some example applications involve extraction of a cardiac monitoring device. The cardiac monitoring device may be an implantable cardiac monitor (ICM) that includes one or more structural and/or functional aspects of the device(s) described in U.S. Patent 9,949,660, entitled, "Method And System To Discriminate Rhythm Patterns In Cardiac Activity".

At least some of the example applications involve extraction of a subcutaneous IMD (S-IMD) that is configured to be implanted in a subcutaneous area exterior to the heart. In at least one example, the S-IMD may be an ICM that is implanted in a subcutaneous region of the patient. The S-IMD may include one or more structural and/or functional aspects of the device(s) described in U.S. Application Serial No. 17/804,041 (US 2023/0381500), titled "Method And Implantable Medical Device For Reducing Defibrillation Impedance" and filed May 25, 2022; U.S. Patent No. 10,765,860, titled "Subcutaneous Implantation Medical Device With Multiple Parasternal-Anterior Electrodes" and filed May 7, 2018; U.S. Patent No. 10,722,704, titled "Implantable Medical Systems And Methods Including Pulse Generators And Leads" and filed May 7, 2018; and U.S. Patent No. 11,045,643, titled "Single Site Implantation Methods For Medical Devices Having Multiple Leads", filed May 7, 2018. Further, one or more combinations of IMDs may be utilized from the above mentioned patents and applications in accordance with embodiments herein.

The term "subcutaneous" shall mean below the skin, but not intravenous. For example, a subcutaneous device is not located in a chamber of the heart, in a vein on the heart, or in the lateral or posterior branches of the coronary sinus. A subcutaneous device may be located between the skin and the rib cage, or within an intercostal area between two ribs of the rib cage. The rib cage collectively refers to the ribs, sternum, and thoracic vertebrae. Optionally, subcutaneous placement may include the substernal, extra-pericardial space defined between the undersurface of the rib cage and the pericardium or outer portion of the heart.

The term "oblong" shall mean an elongated shape that is longer in at least one dimension than another dimension, such that the oblong shape is not circular/cylindrical or square/cubic. The longest dimension of a cross-sectional shape is referred to herein as a "major dimension," and a shorter dimension of the cross-sectional shape is referred to as a "minor dimension." The minor dimension may be perpendicular to the major dimension.

The term "stadium shape" shall mean an elongated geometric shape having opposite first and second linear lengths and rounded ends between the linear lengths. The rounded ends may be semicircular.

Embodiments set forth herein describe an extraction tool that assists an operator with manually removing an implanted IMD from a patient. The operator may be a physician or other medical professional. The extraction tool may be designed to extricate S-IMDs that are implanted in a subcutaneous region of the patient. For example, the extraction tool may be sized and shaped to accommodate subcutaneously-implanted cardiac monitors. In various examples, the extraction tool has a tray defined by an elongated, thin platform and a raised rim. The size and shape of the platform may be based on the geometry of one or more S-IMDs. The extraction tool has a handle that is connected to the tray and extends from the tray. The operator may hold the handle to manipulate the extraction tool.

For example, the operator may insert at least the tray of the extraction tool into the patient through an incision in the skin. The extraction tool my be relatively thin and narrow to facilitate insertion and advancement through patient tissue with limited tissue trauma. The operator may advance the extraction tool so that the tray moves along a side of the S-IMD and reaches a distal end of the S-IMD. The distal end of the S-IMD may be a portion of the S-IMD that is farthest from the incision (e.g., farthest from the point of entry of the extraction tool). The operator may pivot or otherwise move the handle so that the tray moves toward the S-IMD. The raised rim may capture the S-IMD onto the platform of the tray. The operator may then use the elongated handle to withdraw the extraction tool from the patient with the S-IMD present on the tray. For example, as the extraction tool is pulled in a withdrawing direction out of the patient, the raised rim at a distal edge of the platform may contact the distal end of the S-IMD. The force applied at the contact interface with the distal end of the S-IMD may effectively pull the S-IMD in a proximal direction towards the incision. In various examples, the extraction tool may have a tapered tip at one or both ends of the tool to assist with blunt tissue dissection of the tissue in the subcutaneous pocket in which the S-IMD is located.

The extraction tool may provide several advantages over conventional forceps for removing S-IMDs from patients. In a first technical effect, the extraction tool may reduce the manual forces that are required to extricate the S-IMD compared to conventional forceps. For example, the extraction tool may dissect the S-IMD from some of the surrounding tissue in the subcutaneous pocket as the extraction tool is advanced along the side of the S-IMD toward the distal end of the S-IMD, which reduces tissue encapsulation of the S-IMD. Furthermore, the extraction tool essentially couples to the S-IMD via the raised rim so the S-IMD moves with the tray as the operator pulls the handle in the withdrawing direction. The extraction tool does not rely on gripping opposite sides of the S-IMD to secure the S-IMD to the tool, unlike forceps, so the operator does not have to apply increased force to overcome slippage. Another technical effect may be that the extraction tool is compatible with multiple different IMD form factors, so the extraction tool can be used to extract multiple different IMDs. Furthermore, the extraction tool may be relatively thin and compact to limit discomfort of the patient during the extraction process. Another technical effect may be that the extraction tool is reusable, rather than single-use, to increase the utility and/or value of the tool. For example, the extraction tool may be sterilizable. These and other advantages are described in more detail herein with reference to the figures.

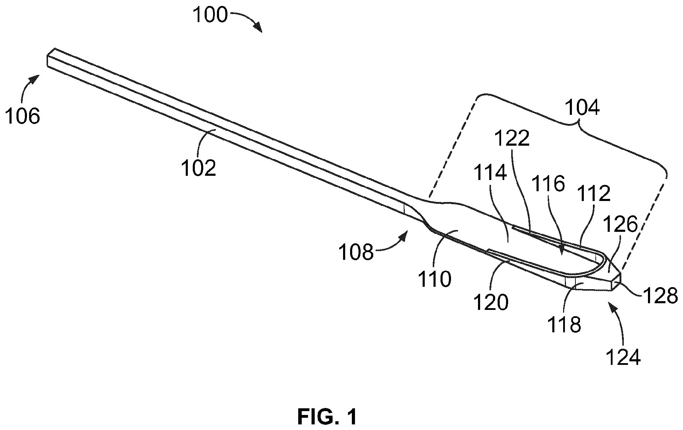

Figure 1 illustrates an extraction tool 100 formed in accordance with an example embodiment. The extraction tool 100 includes a handle 102 and a tray 104 that extends from the handle 102. The tray 104 is designed to be inserted into a patient to capture an S-IMD for removing the S-IMD from the patient as the extraction tool 100 is withdrawn. In an example, the extraction tool 100 has a unitary or one-piece (e.g., monolithic) construction such that the tray 104 is seamlessly connected to the handle 102. The extraction tool 100 may have a metal composition and/or a plastic composition. For example, the extraction tool 100 may be stamped and formed from a thin metal sheet, may be an injection-molded rigid plastic, may be additively manufactured (e.g., 3D printed), or the like.

The handle 102 is elongated and extends from a proximal end 106 of the handle 102 to a distal end 108 of the handle 102. The tray 104 is located at the distal end 108. The handle 102 is designed to be grasped by the hand of an operator. The handle 102 may have a relatively thin and narrow shape, at least proximate to the distal end 108 which may be advanced through an incision in a patient. In the illustrated example, the handle 102 has an approximately uniform thickness along the entire length. In another example, the handle 102 may have a greater thickness at the proximal end 106 than the distal end 108. A segment of the handle 102 at the proximal end 106 may be thicker to enhance comfort of the operator that holds that segment by hand. The handle 102 in Figure 1 is approximately linear. The linear orientation of the handle 102 may assist the operator with maneuvering the tray 104 through the patient anatomy and towards a side of the S-IMD. In another example, however, the handle 102 may have a curvature along at least a segment of the handle 102.

The tray 104 has a platform 110 and a raised rim 112. The platform 110 may be flat, at least along a top surface 114 of the platform 110. The platform 110 may be wider (e.g., broader) than the distal end 108 of the handle 102. The width of the platform 110 may be based on the width of one or more S-IMDs. For example, the width of the platform 110 may be at least slightly greater (e.g., 5%, 10%, or the like) than the width of a housing of an S-IMD to accommodate and support the S-IMD on the platform 110.

The raised rim 112 projects above the top surface 114 of the platform 110. The top surface 114 of the platform 110 and the raised rim 112 define a cavity 116 designed to receive and contain the S-IMD therein. The raised rim 112 at least partially surrounds the top surface 114. In the illustrated example, the raised rim 112 is located at a distal edge 118 of the platform 110 and curves to extend from the distal edge 118 along both a first side edge 120 and a second side edge 122 of the platform 110. The first and second side edges 120, 122 are opposite one another along the width of the platform 110. In Figure 1, the raise rim 112 extends approximately half the length of each of the side edges 120, 122. The cavity 116 may be located along the distal portion of the tray 104 that includes the raised rim 112. When the S-IMD is present in the cavity 116, a portion of the S-IMD may extend along the platform 110 on a proximal portion of the tray 104 that does not include the raised rim 112. In the illustrated example, the height of the raised rim 112 varies along the length of the tray 104. For example, the raised rim 112 is tallest at the distal edge 118 and tapers in height along the side edges 120, 122 with increasing distance from the distal edge 118. In another example, the height of the raised rim 112 may be uniform along the length of the tray 104.

In Figure 1, a distal end 124 of the tray 104 has a tapered tip 126. The distal end 124 of the tray 104 may represent the distal end of the extraction tool 100. For example, the platform 110 and raised rim 112 may be located between the handle 102 and the tapered tip 126. The tapered tip 126 may be the first portion of the extraction tool 100 that is inserted into the patient through an incision. The tapered tip 126 may provide a leading or front end of the tool 100. The tapered tip 126 has a tapered shape to assist with tunneling the extraction tool 100 through the patient tissue to the implanted S-IMD. For example, the tapered shape may reduce the risk of the tray 104 snagging on tissue or tearing tissue as the tray 104 is advanced toward the S-IMD. In an example, the tapered tip 126 may have a blunt end 128. For example, the tip 126 may taper along its length until the blunt end 128, which is a relatively flat surface. The blunt end 128 may provide blunt dissection of the patient tissue from the S-IMD as the extraction tool 100 tunnels toward a distal end of the S-IMD.

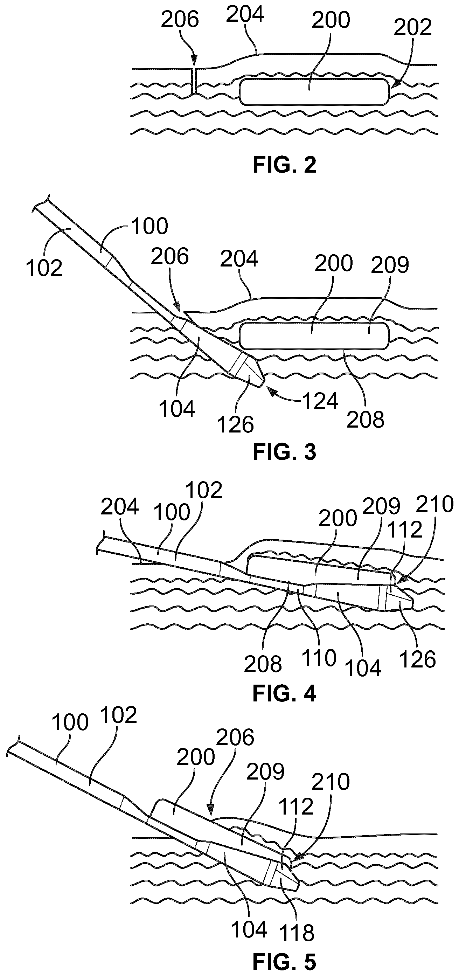

Figures 2 through 5 illustrate a sequence of events during an extraction procedure in which the extraction tool 100 is used to remove an S-IMD 200 from a subcutaneous region of a patient. Figures 2 through 5 depict the extraction tool 100 of Figure 1, but the other example embodiments of the extraction tool 100 described herein can be used to perform the same or a similar extraction procedure.

Figure 2 shows an S-IMD 200 implanted in a subcutaneous pocket 202 of a patient below the skin 204 of the patient. To remove the S-IMD 200, an operator may make an incision 206 in the skin 204 near the S-IMD 200 using a scalpel or another cutting instrument.

Figure 3 shows a portion of the extraction tool 100 at a first advancement position as the extraction tool 100 is inserted through the incision 206 into the patient. The operator may hold the handle 102 and use the handle 102 to press the tray 104 through the incision 206 with the tapered tip 126 at the distal end 124 leading. The tapered tip 126 may perform blunt dissection of tissue as the extraction tool 100 tunnels through the patient. The operator may manipulate the handle 102 to control the trajectory of the tray 104. In an example, the operator may direct the extraction tool 100 for the tray 104 to move along an underside 208 of a housing 209 of the S-IMD 200. The underside 208 of the housing 209 is the side that faces internally, in an opposite direction from the skin 204.

Figure 4 shows the extraction tool 100 at a second advancement position as the extraction tool 100 is moved along the underside 208 of the S-IMD 200. The operator may control the extraction tool 100 for the tray 104 to move along the underside 208 of the S-IMD 200 towards a distal end 210 of the housing 209 of the S-IMD 200. The operator may reduce the angle the extraction tool 100 relative to the skin 204 to permit tunneling in a more lateral direction than when initially inserting the tray 104 through the incision 206, as shown in Figure 3. The tapered tip 126 may continue to dissect the tissue and assist with extricating the S-IMD 200 from the surrounding tissue as the extraction tool 100 is advanced.

Once the raised rim 112 at the distal edge 118 of the platform 110 passes beyond the distal end 210 of the housing 209, the housing 209 may be received into the cavity 116 (shown in Figure 1) of the tray 104. For example, the operator may use the handle 102 to pivot the extraction tool 100 so the tray 104 moves toward the skin 204. The S-IMD 200 may be captured by the tray 104 when the housing 209 is received into the cavity 116.

Figure 5 shows the extraction tool 100 at a withdrawal position as the extraction tool 100 is being removed from the patient. The operator may use the handle 102 to pull the extraction tool 100 in a withdrawing direction out of the patient through the incision 206. The movement of the extraction tool 100 moves the S-IMD 200 toward the incision 206. For example, the S-IMD 200 is maintained in the cavity 116 of the tray 104 as the extraction tool 100 is pulled out of the patient. The raised rim 112 at the distal edge 118 may abut the distal end 210 of the housing 209. The force applied at the contact interface between the raised rim 112 and the distal end 210 of the housing 209 may effectively pull the S-IMD 200 in a proximal direction towards the incision 206 as the extraction tool 100 is withdrawn. The force applied by the raised rim 112 frees the S-IMD 200 from any remaining tissue encapsulation. The S-IMD 200 may be disposed on the tray 104 as the tray 104 is withdrawn from the patient through the incision 206.

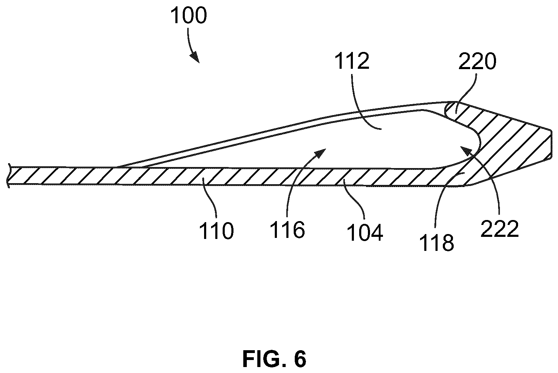

Figures 6 through 11 show example variations of the extraction tool 100 shown in Figures 1 through 5. The extraction tools 100 shown in Figures 6 through 11 may have one or more different features than the extraction tool 100 in Figure 1, but may be used by an operator to extract an implanted S-IMD via the same or a similar process as described with reference to Figures 2 through 5. Unless explicitly noted, the features of the different example embodiments shown in Figures 1 and 6 through 11 may be combined to represent additional embodiments that are within the scope of this disclosure.

Figure 6 is a cross-sectional view of a portion of the tray 104 of the extraction tool 100 according to another example embodiment. The cross-section plane may bifurcate the extraction tool 100 along the longitudinal length of the tool 100. In the extraction tool 100 shown in Figures 1 through 5, for example, the raised rim 112 at the distal edge 118 of the platform 110 may be a lip or wall that generally extends perpendicular to the plane of the top surface 114 of the platform 110. For example, the raised rim 112 in Figures 1 through 5 may be generally linear in the height dimension. In the example shown in Figure 6, however, the raised rim 112 at the distal edge 118 of the platform 110 is curved along the height of the raised rim 112 to form a shelf 220 that at least partially overhangs the platform 110. The shelf 220 may hook onto the distal end 210 of the housing 209 to enhance the capture of the S-IMD 200 during the extraction process. For example, the shelf 220 and the platform 110 define a pocket 222, which is an extension of the cavity 116. The pocket 222 may be sized and/or shaped to receive the distal end 210 of the housing 209 of the S-IMD 200 therein, or a portion of the distal end 210 of the housing 209.

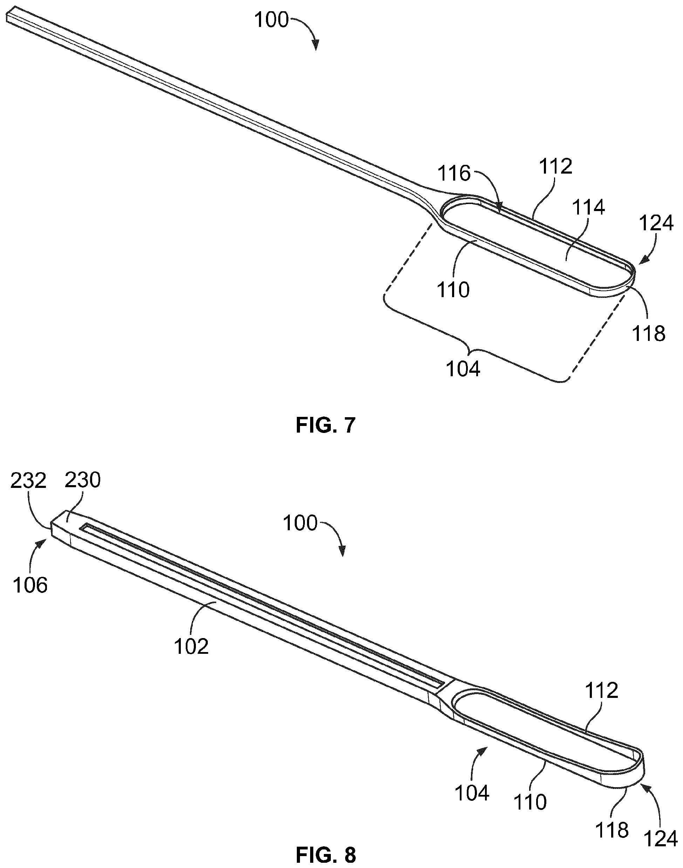

Figure 7 shows the extraction tool 100 according to an example embodiment in which the raised rim 112 of the tray 104 fully surrounds the top surface 114 of the platform 110. For example, the raised rim 112 extends around a full perimeter edge of the platform 110. The cavity 116 of the tray 104 that receives the S-IMD has a closed perimeter defined by the raised rim 112. The shape of the cavity 116 may be oblong. In an example, the size and shape of the cavity 116 may be based on the size and shape of the housing 209 of the S-IMD 200. For example, the length dimension, the width dimension, and the surface area of the top surface 114 within the cavity 116 may be selected to accommodate at least one type of S-IMD. The dimensions may be slightly larger than the known dimensions of the S-IMD(s) to permit the tray 104 to capture the S-IMDs during the extract procedure without difficulty. The oblong shape of the cavity 116 shown in Figure 7 may be selected to accommodate S-IMDs that have oblong-shaped housings. In an example, the cavity 116 has a stadium (e.g., racecourse) shape defined by the raised rim 112.

In Figure 7, the distal end 124 of the tray 104 is rounded but does not have a tapered tip that performs blunt dissection. Optionally, the raised rim 112 may have a uniform or constant height along the length of the raised rim 112 surrounding the cavity 116. In another example, a portion of the raised rim 112 may have a greater height than another portion of the raised rim 112. For example, the raised rim 112 at the distal edge 118 of the platform 110 may be taller than a portion of the raised rim 112 spaced apart from the distal edge 118.

Figure 8 shows the extraction tool 100 according to another example embodiment in which the handle 102 includes a tapered tip 230. The tapered tip 230 is located at the proximal end 106 of the handle 102, opposite the tray 104. The tapered tip 230 has a blunt end 232 for blunt dissection of patient tissue from the S-IMD. In the illustrated example, the extraction procedure may be modified so that the operator first inserts the proximal end 106 of the handle 102 into the patient. The tapered tip 230 may lead and assist with tunneling through the patient tissue, as well as dissecting the tissue from the S-IMD. The handle 102 may be thicker and/or wider than the handles shown in Figures 1 and 7 to define a tunnel that has sufficient width and height to accommodate the tray 104. Once a tunnel is formed, the operator may remove the extraction tool 100 from the patient, sanitize the tool 100 (or select a different, sanitized extraction tool 100), and reenter the patient with the distal end 124 of the tray 104 leading. The operator uses the handle 102 to maneuver the tray 104 along the tunnel path that was created by the handle 102 to reach the distal end of the S-IMD and capture the S-IMD on the tray 104.

The tray 104 of the extraction tool 100 in Figure 8 is similar to the tray 104 in Figure 7, except that the height of the raised rim 112 is not uniform along the length. For example, the raised rim 112 at the distal edge 118 of the platform 110 is taller than a portion of the raised rim 112 spaced apart from the distal edge 118.

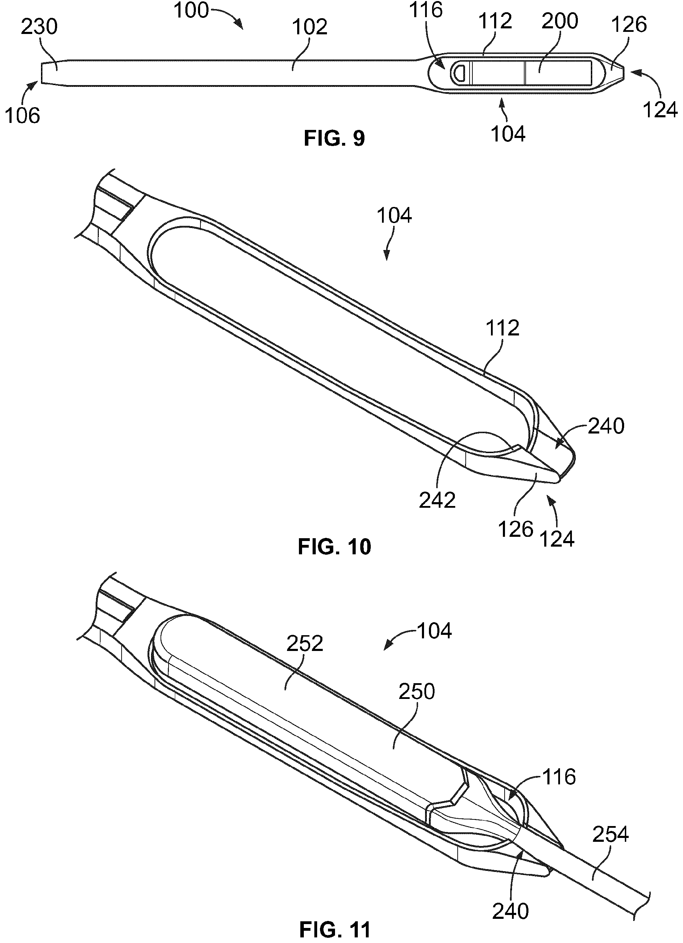

Figure 9 shows the extraction tool 100 according to an example embodiment in which the raised rim 112 fully surrounds the S-IMD 200 disposed within the cavity 116. The extraction tool 100 in Figure 9 is a combination of features from the extraction tools 100 shown in Figures 1, 7, and 8. For example, the illustrated extraction tool 100 may have the same handle 102 as shown in Figure 8, with the tapered tip 230 at the proximal end 106. The cavity 116 is oblong and is fully surrounded by the raised rim 112, similar to the examples in Figures 7 and 8. The tray 104 in Figure 9 has a tapered tip 126 at the distal end 124, similar to the example shown in Figure 1. The extraction tool 100 in Figure 9 includes tapered tips 126, 230 at both ends.

Figure 10 shows the tray 104 of the extraction tool 100 according to an example embodiment in which the raised rim 112 defines a depression 240 at the distal end 124 of the tray 104. The depression 240 may be a groove or channel that is open at a top edge 242 of the raised rim 112. The depression 240 may continue through the tapered tip 126 to the distal end 124. The depression 240 in the raised rim 112 is designed to accommodate a projection that extends from a housing of the S-IMD.

For example, Figure 11 shows the tray 104 of Figure 10 with an S-IMD 250 located in the cavity 116. The S-IMD 250 has a housing 252 that is oblong and is positioned on the top surface 114 of the platform 110. The S-IMD 250 has a projection 254 that extends from a distal end of the housing 252. The projection 254 may be a lead, an antenna, a fixation element for anchoring the S-IMD 250 at an implant location, or the like. The projection 254 extends through the depression 240. Without the depression 240, the projection 254 may contact the raised rim 112 and prohibit the tray 104 from fully and reliably capturing the S-IMD 250 in the cavity 116.

In some embodiments, the extraction tool 100 includes an indicator device for position assurance. The indicator device on the extraction tool 100 may provide an indication to the operator regarding whether or not the S-IMD is aligned with and/or received within the cavity 116 of the tray 104. As shown in Figures 3 and 4, the S-IMD 200 is under the skin 204 of the patient and therefore obstructed from view of the operator as the operator manipulates the extraction tool 100 to move the tray 104 towards the S-IMD 200. The indicator device on the extraction tool 100 may assist the operator by providing sensory feedback, to the operator outside of the patient, indicating a relative position of the tray 104 to the S-IMD 200. The sensory feedback may be visual via a light or laser beam, auditory via an audio emitter, or the like.

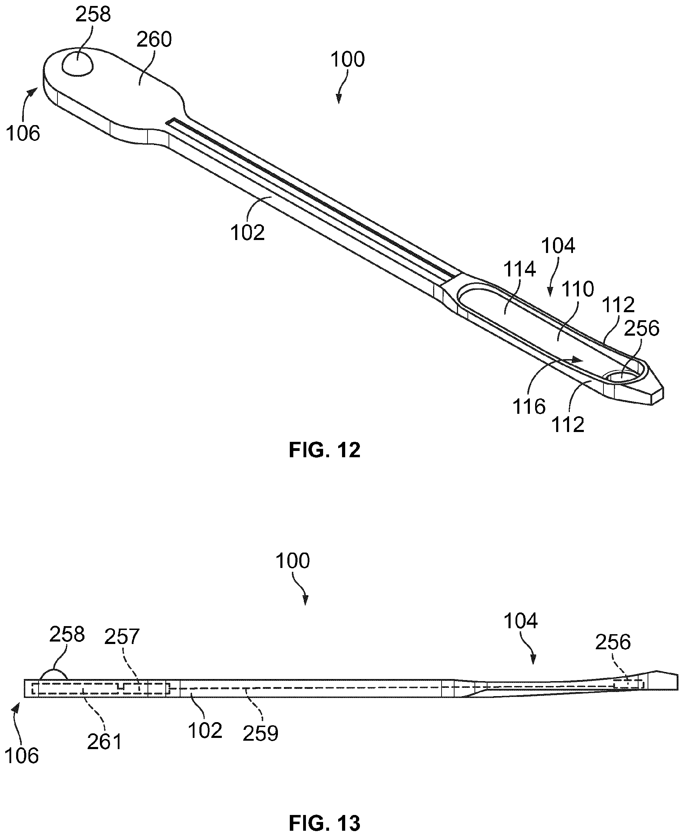

Figure 12 is an elevation view of the extraction tool 100 according to a first indicator embodiment. Figure 13 illustrates a side view of the extraction tool 100 according to the first indicator embodiment. The extraction tool 100 has a force sensor 256 on the tray 104, located on the platform 110. The force sensor 256 is exposed along the top surface 114, and radially interior of the raised rim 112. The force sensor 256 may be a pressure sensor or the like. The force sensor 256 is positioned so that the S-IMD contacts the force sensor 256 when received into the cavity 116 of the tray 104 and contacts the platform 110.

An electronics module 257 is electrically connected to the force sensor 256. The electronics module 257 may be integrated within the extraction device 100. For example, the electronics module 257 may be disposed within the handle 102. The electronics module 257 may include a microprocessor (or equivalent control circuitry), one or more processors, RAM and/or ROM memory, logic and timing circuitry, state machine circuitry, and/or I/O circuitry. The electronics module 257 is electrically connected to the force sensor 256 via a conductive pathway 259 through the body of the tool 100. The conductive pathway 259 may be defined by one or more wires, circuit traces, or the like. The electronics module 257 may include a driver for controlling an indicator device 258 of the extraction tool 100.

The indicator device 258 may be disposed on the handle 102. In an example, the indicator device 258 may be located at or proximate to (e.g., within 2 cm of) the proximal end 106. The indicator device 258 is electrically connected to the electronics module 257 via a conductive pathway, such as a wire, conductive trace, or the like. In a first example, the indicator device 258 is a light source that emits light. The light source may be an LED light emitter. In a second example, the indicator device 258 is an audio emitter, such as a small audio speaker, that outputs sound. In a third example, the indicator device 258 includes a vibration motor, such as a linear resonant actuator (LRA), that vibrates the extraction tool 100. In another example, the extraction tool 100 can include multiple indicator devices 258, such as a light source and a vibration motor, a light source and an audio emitter, all three of the light source, vibration motor, and audio emitter, or the like.

The electronics module 257 may receive sensor signals generated by the force sensor 256. In response to a sensor signal indicating that an S-IMD is received in the cavity 116 of the tray 104 on the platform 110, the electronics module 257 may activate the indicator device 258 to generate an output that notifies the operator that the S-IMD is present (e.g., captured) on the tray 104. The electronics module 257 may determine that an S-IMD is present on the tray 104 when a sensor signal generated by the force sensor 256 indicates that the pressure or force exerted on the force sensor 256 exceeds a designated threshold value. The designated threshold value may be derived or based on the weight of the S-IMD. If the force exerted on the sensor 256 is less than the threshold value, then the electronics module 257 determines that no S-IMD is present on the tray 104, or at least is not correctly positioned on the tray 104 within the cavity 116 to permit a reliable extraction of the S-IMD.

In an example in which the indicator device 258 is the light source, the electronics module 257 may control the light source to not emit any light unless the sensor signals from the force sensor 256 indicate that the S-IMD is present on the tray 104. Once the force sensor 256 detects the weight of the S-IMD exerted on the platform 110, the electronics module 257 controls the light source to emit light. The operator may observe the light to determine that the S-IMD is successfully received in the cavity 116 of the tray 104. After observing the light, the operator may pull the extraction tool 100 in the withdrawing direction out of the patient, assured that the S-IMD will be extracted with the tool 100, as shown and described with reference to Figure 5. In another example in which the indicator device 258 is an audio emitter, the electronics module 257 may control the audio emitter to emit a sound to alert the operator in response to the electronics module 257 detecting, via the force sensor 256, that the S-IMD is on the tray 104. In another example in which the indicator device 258 includes a vibration motor, the electronics module 257 may control the vibration motor (or driver thereof) to vibrate in response to detecting that the S-IMD is on the tray 104, which provides haptic feedback to the operator holding the handle 102.

The handle 102 in the illustrated embodiment has a broad paddle-shaped segment 260 at the proximal end 106 referred to as a paddle segment 260. The paddle segment 260 is wider than the portion of the handle 102 between the paddle segment 260 and the tray 104. The increased width of the paddle segment 260 may provide increased volume and area for housing a power source 261 and/or the electronics module 257 which enable the operations of the force sensor 256 and the indicator device 258. For example, the power source 261 may be one or more batteries that supply electrical energy to the electronics module 257, the force sensor 256, and the indicator device 258. The paddle segment 260 in Figures 12 and 13 is shown as having the same thickness (in the height dimension) relative to the other portion of the handle 102 extending to the tray 104. In another example, the paddle segment 260 may be thicker (e.g., taller) than the other portion of the handle 102 to accommodate the indicator device 258, the power source 261, the electronics module 257, and/or the like.

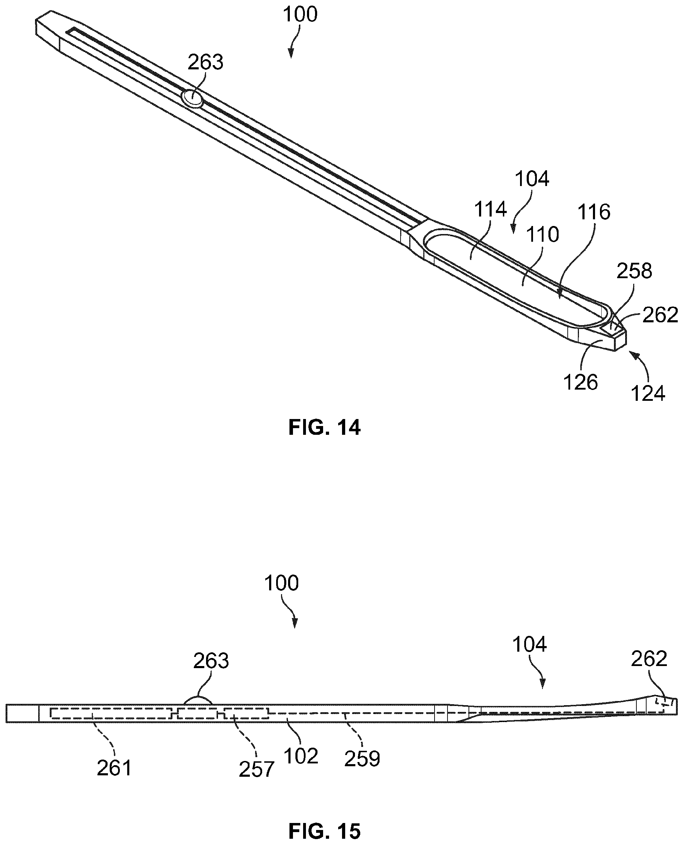

Figure 14 is an elevation view of the extraction tool 100 according to a second indicator embodiment. Figure 15 illustrates a side view of the extraction tool 100 according to the second indicator embodiment. The extraction tool 100 in the illustrated embodiment has an indicator device 258 that is a distal, upward-facing light source 262. The distal light source 262 is located distal of the platform 110. For example, the distal light source 262 may be coupled to the tapered tip 126 at the distal end 124 of the extraction tool 100. The distal light source 262 is positioned to emit light in an upward direction, which corresponds to the general direction that is normal to the top surface 114 of the platform 110 and extends from the top surface 114 towards the open top of the cavity 116. The distal light source 262 may be a surface mount LED. The distal light source 262 may be controlled to emit constant, continuous light during the extraction process.

When the distal portion of the extraction tool 100 is within the patient moving towards the S-ICM, the operator may be able to see the light illuminating a portion of the patient's skin. The light emitted by the distal light source 262 may be obscured by the S-ICM while the tapered tip 126 is under the S-IMD, as shown in Figure 3 for example. Once the tapered tip 126 moves fully beyond the S-IMD, the S-IMD may align with the tray 104 and be received within the cavity 116 thereof. At that stage, the light emitted by the distal light source 262 may once again be visible to the operator because the S-IMD is no longer obstructing or blocking the light from illuminating the skin. The reemergence of the light provides an indication to the operator that the S-IMD has been captured within the tray 104, so the operator can begin pulling in the withdrawing direction. As such, the distal light source 262 provides position assurance that the distal tapered tip 126 of the tool 100 has passed beyond the far end of the S-IMD to reliably capture the S-IMD into the cavity 116 of the tray 104.

In an example, power and control elements for the distal light source 262 are integrated into the handle 102 of extraction tool 100, similar to the embodiment shown in Figure 13. For example, the distal light source 262 may be powered by the power source 261, such as one or more battery cells, disposed within the handle 102 of the extraction tool 100. The distal light source 262 may be electrically connected via the conductive path 259 to the electronics module 257. The electronics module 257 may control and/or drive the operation of the distal light source 262 to generate and emit light.

The electronics module 257 may be electrically connected to an input switch 263. The input switch 263 may include a button, tab, or the like that is exposed along the handle 102. The operator may selectively actuate the input switch 263 to activate and/or deactivate the distal light source 262. For example, the operator may use the input switch 263 to turn the distal light source 262 OFF, so that the distal light source 262 does not emit light, while the extraction tool 100 is not being used to extract an S-IMD. During an extraction procedure, the operator may actuate the input switch 263 to turn the distal light source 262 ON, and then may turn the distal light source 262 OFF again after the S-IMD is successfully withdrawn from the patient.

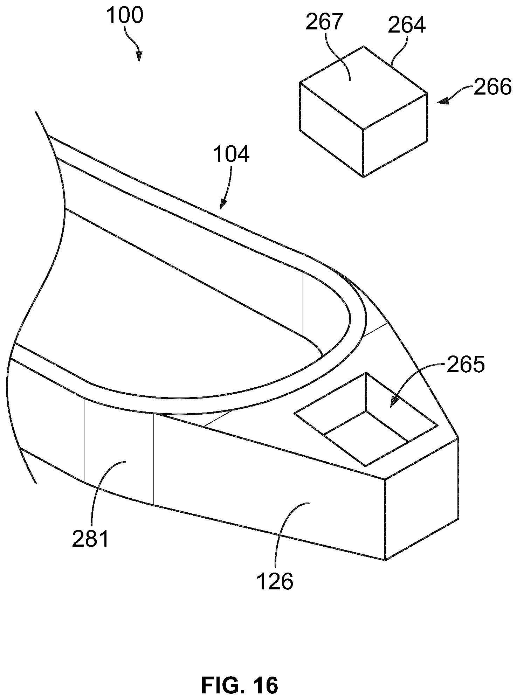

Figure 16 is an exploded elevation view of a distal portion of the extraction tool 100 according to a third indicator embodiment that is a variation of the second indicator embodiment shown in Figures 14 and 15. In the illustrated embodiment, the extraction tool 100 is configured to be coupled to a distal light source 264 that operates in the same way as the distal light source 262 shown in Figures 14 and 15. The difference is that the distal light source 264 in Figure 16 is a self-contained package that is discrete from the body 281 of the extraction tool 100, so can be relatively easily plugged into and removed from the extraction tool 100. The power and control elements of the light source 264 are not integrated into the handle 102 of the extraction tool 100. Instead, the power and control elements are packaged with the light generation element 267 in a single module 266 or stack. For example, the control and power elements may be disposed below the light generation element 267 in the module 266 so that the light generation element (e.g., LED) can emit light in the upward-facing direction as described with reference to Figures 14 and 15.

The distal portion of the tray 104 may define a compartment 265 that is sized and shaped to receive and hold the distal light source 264 (e.g., the module 266). In an example, the compartment 265 is located on the tapered tip 126. The extraction tool 100 may be assembled by bonding the module 266 within the compartment 265 using a medical grade adhesive, or the like. Once assembled, the distal light source 264 operates similar to the distal light source 262 of Figures 14 and 15 to provide assurance that the distal portion of the extraction tool 100 has moved past the end of the S-IMD during an extraction process. A benefit of using the modular distal light source 264 is that the extraction tool 100 may be formed via a relatively simple and cost-effective molding process. The manufacturing process may be relatively simple because the handle 102 does not need to accommodate electronics. The extraction tool 100 itself could be a single-use component that is disposed after every use. The modular distal light source 264 could be removed and reused for additional extraction procedures after sanitization.

In another example, the second indicator embodiment shown in Figures 14 and 15 can allow for selectively coupling the electronics to the extraction tool 100, to achieve similar benefits as the embodiment described with reference to Figure 16. For example, the electronic components may be external and selectively coupled to (and uncoupled from) the body 281 of the extraction tool 100 as desired. The handle 102 may be formed to define one or more openings sized and shaped to accommodate the battery 261, switch 263, and electronics module 257 therein for enabling non-permanent assembly of the extraction tool 100 with indicator functionality. There may be a single cavity or depression along the underside of the handle 102 that commonly holds the battery 261, the switch 263, and the electronics module 257, which are secured to one another as a block or chain. In another example, the handle 102 may include a first opening sized and shaped to hold the battery 261, a second opening sized and shaped to hold the switch 263, and a third opening sized and shaped to hold the electronics module 257. The handle 102 may be formed to include small tabs or detents extending into the one or more openings for selectively coupling the components to the handle 102. In addition, the handle 102 may be formed to include a channel or groove extending along the underside of the handle 102. The channel accommodates the conductive path 259 (e.g., wire(s)) extending from the electronics module 257 to the distal light source 262. In this example embodiment, the body 281 of the extraction tool 100 can be formed via a molding process to include the one or more openings (e.g., cavities, channels, grooves, depressions, etc.) designed to accommodate the electronics components used to provide an indication of capture of the S-IMD. An operator can use the extraction tool 100 with or without the electronic components. If the indicator functionality is desired, the electronics module 257 (and associated components) can be loaded into the corresponding openings in the handle 102. For example, the electronics module 257 may couple to the handle 102 via a snap-fit arrangement using one or more detents or tabs.

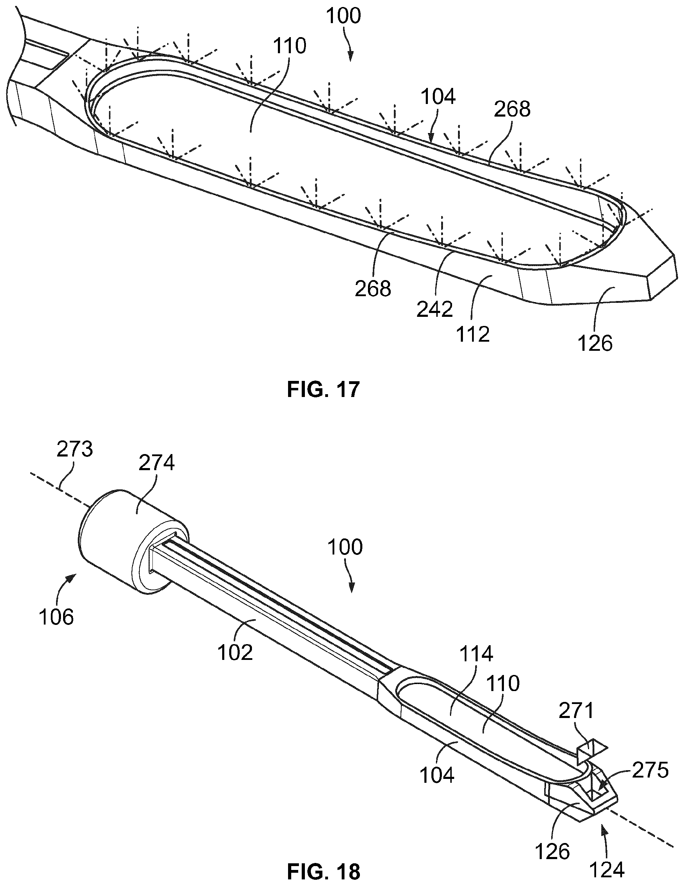

Figure 17 is an elevation view of a distal portion of the extraction tool 100 according to a fourth indicator embodiment that is another variation of the second indicator embodiment shown in Figures 14 and 15. In Figure 17, the extraction tool 100 includes a distal light source 268 that is integrated into the extraction tool 100 similar to the distal light source 262 shown in Figures 14 and 15. However, rather than being a point source that is located on the tapered tip 126, the distal light source 268 is an elongated light strip that at least partially surrounds the platform 110 of the tray 104. In the illustrated embodiment, the distal light source 268 extends along a full perimeter of the tray 104, fully surrounding the platform 110. For example, the distal light source 268 may be disposed at the top edge 242 of the raised rim 112.

The distal light source 268 may be controlled and powered in the same way as the distal light source 262 described with reference to Figures 14 and 15. By extending fully or partially along a perimeter of the tray 104, the distal lights source 268 can provide an outline indicator to the operator during the S-IMD extraction procedure to assist with locating and aligning the S-IMD with the tray 104. For example, the light emitted from the distal light source 268 may appear as a hollow, closed shape illuminated on the patient's skin as the tray 104 is advanced towards the implanted S-IMD. The closed shape may be oblong, as shown in Figure 17. For example, the operator may see an oblong ring of light on the patient's skin. As the tray 104 passes under the S-IMD, the light emitted from different parts of the distal light source 268 gets obstructed or blocked by the S-ICD, which provides an indication of the tray's 104 position relative to the S-IMD. As an example, if the light emitted from the distal and proximal ends of the light strip is visible but only a right strip of light is visible, then that indicates to the operator that the S-IMD is covering the left strip of the light source 268. The operator would then move the extraction tool 100 to the left until the left strip of light is also visible. The perimeter lighting effect provides position assurance that the tray 104 is aligned with the S-IMD in 360 degrees.

In other examples, the distal light source 268 may not continuously extend the full perimeter of the tray 104 as shown in Figure 17. For example, the distal light source 268 may include four discrete point light sources or light strips with one at a distal end of the tray 104, one at a proximal end of the tray 104, one at a left side edge of the tray 104, and the last one at a right side edge of the tray 104. The four lights may be positioned at the top edge 242 of the raised rim 112. When an S-IMD is captured on the tray 104, light emitted from each of the four lights may be visible along the skin of the patient, indicating that capture is achieved.

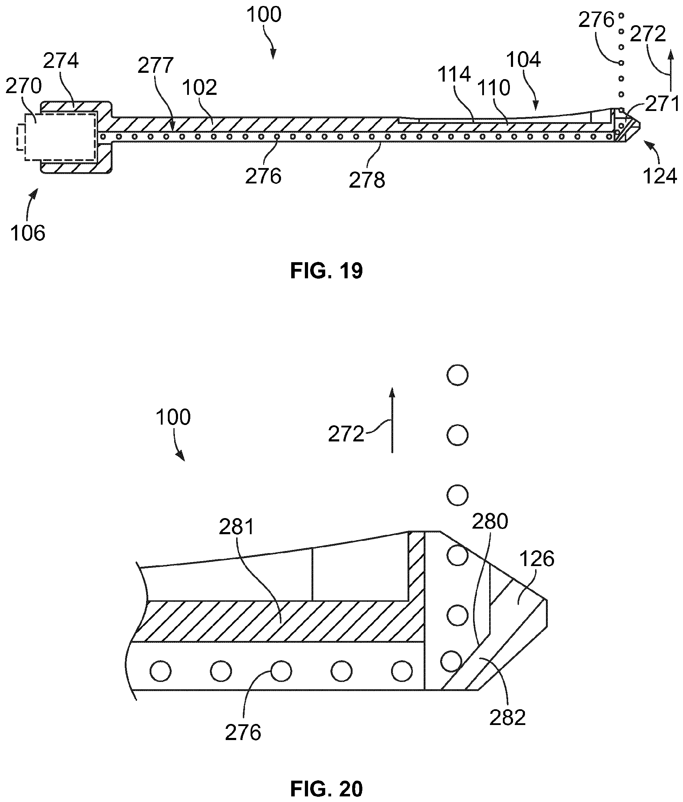

Figure 18 is an exploded, elevation view of the extraction tool 100 according to a fifth indicator embodiment. Figure 19 is a side cross-sectional view of the extraction tool 100 shown in Figure 18. The cross-section is taken along a line that bisects the extraction tool 100 along a length (e.g., longitudinal) axis 273 of the tool 100 that extends from the proximal end 106 to the distal end 124. The indicator device in Figures 18 and 19 includes a laser device 270. The laser device 270 may be mounted to the proximal end 106 of the handle 102. For example, the proximal end 106 of the handle 102 may include a socket 274 with a cavity that is sized to accommodate at least a length of the laser device 270. In an example, the laser device 270 may be a laser pointer that includes a laser light generator, power source (e.g., battery), and switch all contained within a housing of the laser device 270.

The indicator device also includes a beam deflector element 271. The beam deflector element 271 may be mounted to the tapered tip 126 at the distal end 124 of the extraction tool 100. The beam deflector element 271 is shown suspended above the tapered tip 126 in Figure 18. The extraction tool 100 may be assembled by mounting the laser device 270 to the socket 274 and securing the beam deflector element 271 within a recess 275 defined in the tapered tip 126. For example, the beam deflector element 271 may be bonded to the tapered tip 126 via a medical grade adhesive. The beam deflector element 271 is positioned to reflect and/or refract laser light (e.g., a laser beam) 276 emitted by the laser device 270 in an upward direction 272 that is normal to the top surface 114 of the platform 110. The beam deflector element 271 may include or represent one or more prisms, mirrors, and/or the like. In an example, the beam deflector element 271 is a 90-degree prism.

During an extraction procedure, the laser device 270 may be activated to emit laser light 276 along a length of the extraction tool 100 from the laser device 270 to the beam deflector element 271. In an example, the extraction tool 100 may define a channel 277 that continuously extends along the handle 102 and the tray 104. The channel 277 in the illustrated example is a groove that is defined along a bottom side 278 of the extraction tool 100 and is open at the bottom of the extraction tool 100. In another example, the channel 277 may be a closed tunnel that is within an interior of the body of the extraction tool 100, along both the handle 102 and the tray 104. The beam deflector element 271 may deflect the laser light (e.g., laser beam) 276 approximately 90 degrees so that the laser light 276 travels generally in the upward direction 272 upon exiting the beam deflector element 271.

This embodiment shown in Figures 18 and 19 may function similar to the distal light source embodiments shown in Figures 14 through 17. For example, when the tray 104 is disposed within a patient during an extraction procedure, the laser device 270 may be active to emit laser light 276 that impinges on the beam deflector element 271 and is emitted in the upward direction 272. The laser light 276 may be visible to the operator by illuminating the skin of the patient, unless the light beam or path is obstructed by the S-IMD while the tray 104 is under the S-IMD. The reemergence of the laser light 276 illuminating the skin may indicate to the operator that the beam deflector element 271 at the tapered tip 126 has passed the far end of the S-IMD, so that the S-IMD is likely captured on the tray 104. One or more technical benefits of this embodiment are based on the laser device 270 being an external source that is selectively coupled to and removed from the extraction tool 100. Similar to the discrete light module 266 shown in Figure 16, the body 281 of the extraction tool 100 can be formed via a relatively simple process without having to integrate electronics and power sources into the body 281. The body 281 is the physical material of the tool 100 that forms the handle 102, the tray 104, and the tapered tip 126.

Figure 20 illustrates a cross-sectional view of a distal portion of the extraction tool 100 according to a sixth indicator embodiment. The cross-section in Figure 20 may bifurcate the extraction tool 100 along the longitudinal length of the tool 100, similar to Figure 19. The embodiment of Figure 20 is a variation of the embodiment shown in Figures 18 and 19. The extraction tool 100 includes a beam deflector element 280 that has the same function as the beam deflector element 271 shown in Figures 18 and 19, but is integrated as part of the body 281 of the extraction tool 100. For example, the beam deflector element 280 may be a reflective surface of an angled wall 282 of the body 281. The angled wall 282 is positioned and angled so that the laser light 276 impinges on the beam deflector element 280 and is emitted in the upward direction 272.

The angled wall 282 may be an integral part of the body 281, such that the angled wall 282 is formed while the body 281 is being manufactured. In an example, the angled wall 282 may be molded during a molding process that forms the body 281. The beam deflector element 280 in an example is a polished (e.g., smooth) surface of the angled wall 282. In another example, the beam deflector element 280 may include a planar reflective element, such as a mirror layer, a reflective sticker, or the like. The planar reflective element may be affixed to the angled wall 282, such as via an adhesive.

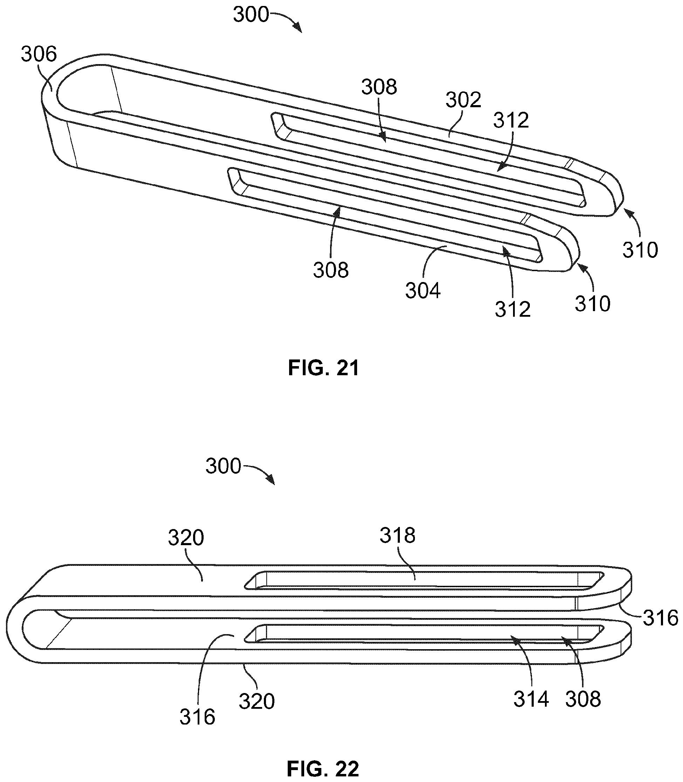

Figure 21 is a hinged extraction tool 300 according to an example embodiment. The hinged extraction tool 300 may be used by an operator to manually extract an IMD from a subcutaneous region of a patient. For example, the hinged extraction tool 300 may be used to perform a similar function as the example extraction tools 100 shown in Figures 1 through 20. The hinged extraction tool 300 may be a handheld instrument that resembles a conventional forceps or tweezers tool with several enhancements to provide better grip on S-IMDs without requiring substantial applied force.

The hinged extraction tool 300 includes a first arm 302 and a second arm 304 that are connected to one another at a hinge 306. The hinged extraction tool 300 may have a unitary, one-piece construction so that the arms 302, 304 are seamlessly connected at the hinge 306. For example, the hinged extraction tool 300 may be stamped and formed from a thin metal sheet, may be an injection-molded rigid plastic, may be additively manufactured (e.g., 3D printed), or the like.

Each of the first and second arms 302, 304 defines a respective channel 308. The two channels 308 may have the same size and shape. The channels 308 may be positioned across from one another in alignment with one another along a length of the hinged extraction tool 300. The channels 308 may be located proximate to respective distal ends 310 of the arms 302, 304. In an example, the channels 308 are near the distal ends 310 but are spaced apart from the distal ends 310. The channels 308 may be elongated along a length of the arms 302, 304. The shape and dimensions of the channels 308 may be selected based on the form factor of one or more known S-IMDs that the tool 300 may be used to extract. For example, the channels 308 may be designed to receive opposite sides of a housing of an S-IMD therein. The housing may extend into the channels 308 of the arms 302, 304, which may increase the grip of the tool 300 on the housing. In the illustrated example, the channels 308 are windows 312 or cutouts that extend fully through a thickness of the arms 302, 304.

Figure 22 illustrates the hinged extraction tool 300 according to a second example hinged embodiment. The hinged extraction tool 300 in Figure 22 may be similar to the example in Figure 21 except that the channels 308 are recesses or grooves 314 along inner surfaces 316 of the arms 302, 304, rather than windows 312. The channels 308 in Figure 22 do not extend fully through the thickness of the arms 302, 304. Optionally, the arms 302, 304 may have protruding mounds 318 along outer surfaces 320 of the arms 302, 304. For example, the grooves 314 may be formed by punching or stamping the arms 302, 304, and the punched or stamped material that is deflected or compressed may form the mounds 318.

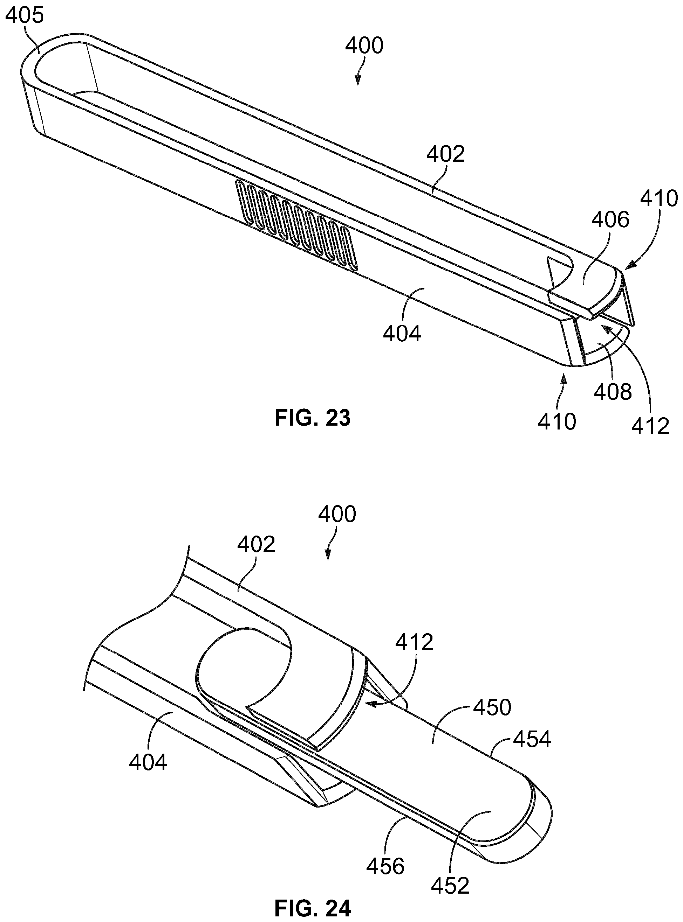

Figure 23 illustrates another hinged extraction tool 400 according to an embodiment. The hinged extraction tool 400 has first and second arms 402, 404 that are joined at a hinge 405, similar to the examples of the hinged extraction tool 300 shown in Figures 21 and 22. The hinged extraction tool 400 is also designed to grasp S-IMDs for extracting the S-IMDs from patients. The hinged extraction tool 400 lacks channels in the arms 402, 404.

Each of the two arms 402, 404 has a respective tab 406, 408 at the distal ends 410 of the arms 402, 404. A first tab 406 extends from the first arm 402, and a second tab 408 extends from the second arm 404. The tabs 406, 408 extend into a gap 409 defined between the arms 402, 404. The tabs 406, 408 may be spaced apart from one another along a height of the tool 400 so that the tabs 406, 408 do not contact each other, even when the arms 402, 404 are pressed together. The tabs 406, 408 and the arms 402, 404 may define a cavity 412 that is designed to receive the housing of an S-IMD therein. The width of the tabs 406, 408 may be selected to control upper and lower limits of the width of the cavity 412. For example, when the arms 402, 404 are pressed towards each other, the first tab 406 may abut the second arm 404 and/or the second tab 408 may abut the first arm 402 to provide a hard stop when the lower limit is reached.

Figure 24 shows a distal section of the hinged extraction tool 400 of Figure 23 gripping an S-IMD 450. The housing 452 of the S-IMD 450 extends into the cavity 412. The operator may press the two arms 402, 404 towards each other to grip opposite sides 454, 456 of the housing 452 to extract the S-IMD 450 from a subcutaneous pocket of the patient.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

The term "approximately" as used herein includes a threshold range of values above and below the stated value. For example, the threshold range may be +/- 5%, 3%, 2%, or the like.

It is to be understood that the above description is intended to be illustrative, and not restrictive. For example, the above-described embodiments (and/or aspects thereof) may be used in combination with each other. In addition, modifications may be made to adapt a particular situation or material to the teachings of the inventive subject matter without departing from its scope. While the dimensions and types of materials described herein are intended to define the parameters of the inventive subject matter, they are by no means limiting and are exemplary embodiments. Many other embodiments will be apparent to one of ordinary skill in the art upon reviewing the above description. The scope of the inventive subject matter should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An extraction tool (100) comprising:
a handle (102); and
a tray (104) that extends from the handle (102) and is configured to be advanced into a subcutaneous region of a patient, the tray (104) including a platform (110) and a raised rim (112) that projects above a top surface (114) of the platform (110), wherein the top surface (114) of the platform (110) and the raised rim (112) define a cavity (116) configured to receive and contain a subcutaneous implantable medical device, S-IMD, (200, 250) therein,
wherein the handle (102) is configured to be manipulated by an operator to withdraw the extraction tool (100) from the patient with the S-IMD (200, 250) on the tray (104).

2. The extraction tool of claim 1, wherein the raised rim (112) is located at a distal edge (118) of the platform (110) and extends along at least a portion of opposite first and second side edges (120, 122) of the platform (110).

3. The extraction tool of claim 1 or 2, wherein the raised rim (112) extends along a full perimeter of the platform (110) and surrounds the top surface (114) of the platform (110).

4. The extraction tool of any one of claims 1 to 3, wherein a distal end (124) of the tray (104) has a tapered tip (126), wherein the platform (110) and the raised rim (112) are located between the handle (102) and the tapered tip (126), optionally wherein the tapered tip (126) has a blunt end (128) for blunt dissection of patient tissue away from the S-IMD.

5. The extraction tool of any one of claims 1 to 4, wherein the tray (104) extends from a distal end (108) of the handle (102), and a proximal end (106) of the handle (110) has a tapered tip (230).

6. The extraction tool of any one of claims 1 to 5, wherein the raised rim (112) has a depression (240) at a distal end (124) of the tray (104) configured to accommodate a projection (254) that extends from a housing (209, 252) of the S-IMD (200, 250).

7. The extraction tool of any one of claims 1 to 6, wherein the raised rim (112) at a distal edge (118) of the platform (110) is curved along a height of the raised rim (112) to form a shelf (220) that at least partially overhangs the platform (110) and defines a pocket (222), the pocket (222) configured to receive a distal end (210) of the S-IMD (200, 250) therein.

8. The extraction tool of any one of claims 1 to 7, further comprising:
a force sensor (256) mounted along the top surface (114) of the platform (110);
an electronics module (257) electrically connected to the force sensor (256) and disposed within the handle (102); and
an indicator device (258) electrically connected to the electronics module (257) and mounted on the handle (102),
wherein, responsive to receiving a sensor signal generated by the force sensor (256) indicating that a force exerted on the force sensor (256) exceeds a designated threshold, the electronics module (257) is configured to activate the indicator device (258) to generate an output that notifies the operator that the S-IMD (200, 250) is on the tray (104), optionally wherein the indicator device (258) includes at least one of a light source that emits light as the output, an audio emitter that generates a sound as the output, or a vibration motor that vibrates the handle as the output.

9. The extraction tool of any one of claims 1 to 8, wherein a distal end (124) of the tray (104) has a tapered tip (126), the extraction tool (100) further comprising a distal light source (262) mounted on the tapered tip (126).

10. The extraction tool of claim 9, further comprising:
an electronics module (257) and a power source (261) that are both disposed within the handle (102) of the extraction tool (100) and electrically connected to the distal light source (262); and
an input switch (263) mounted to the handle (102) and electrically connected to the electronics module (257), the input switch (263) configured to be selectively actuated by the operator to activate and deactivate the distal light source (262).

11. The extraction tool of any one of claims 1 to 10, wherein a distal end (124) of the tray (104) has a tapered tip (126) that defines a compartment (265), the extraction tool (100) further comprising:
a distal light module (266) mounted within the compartment (265), the distal light module (266) including a light generation element (267) configured to emit light.

12. The extraction tool of any one of claims 1 to 11, wherein a distal end (124) of the tray (104) has a tapered tip (126) that defines a compartment (265) configured to receive a distal light source (264) therein, and the handle (102) of the extraction tool (100) defines one or more openings configured to receive at least an electronics module (257) therein, the electronics module (257) configured to control the distal light source (264).

13. The extraction tool of any one of claims 1 to 12, further comprising at least one distal light source (268) mounted to a top edge (242) of the raised rim (112) and configured to emit light, optionally wherein the at least one distal light source (268) is a light strip that extends along a full perimeter of the tray (104).

14. The extraction tool of any one of claims 1 to 13, further comprising:
a laser device (270) mounted to a proximal end (106) of the handle (102); and
a beam deflector element (271) at a tapered tip (126) of the tray (104) that is distal of the platform (110), wherein the beam deflector element (271) is configured to receive laser light emitted by the laser device (270) and redirect the laser light in an upward direction, optionally wherein
the beam deflector element (271) is a prism that is mounted within the tapered tip (126); or
the beam deflector element (271) is a reflective surface on an angled wall (282) of the tapered tip (126).

15. The extraction tool of any one of claims 1 to 14, wherein
the handle (102) includes a proximal end (106) and a distal end (108), wherein the proximal end (106) of the handle (102) has a tapered tip (230); and
the tray (104) extends from the distal end (108) of the handle (102), wherein the raised rim (112) extends along a full perimeter of the platform (110) and surrounds the top surface (114) of the platform (110) to define the cavity (116) therein.
